(19) Europäisches Patentamt
European Patent Office
Office européen des brevets

(11) **EP 0 555 046 B1**

(12) **EUROPEAN PATENT SPECIFICATION**

(45) Date of publication and mention
of the grant of the patent:
**30.07.1997 Bulletin 1997/31**

(51) Int Cl.$^6$: **C12Q 1/48**

(21) Application number: **93300744.5**

(22) Date of filing: **02.02.1993**

(54) **Method for determination of calcium**

Verfahren zur Bestimmung von Calcium

Méthode pour la détermination du calcium

(84) Designated Contracting States:
**DE FR GB IT**

(30) Priority: **07.02.1992 JP 56044/92**

(43) Date of publication of application:
**11.08.1993 Bulletin 1993/32**

(73) Proprietor: **ORIENTAL YEAST CO., LTD.**
**Tokyo (JP)**

(72) Inventors:
 • **Nonobe, Masatsuga**
 **Sanda-shi, Hyogo-ken (JP)**
 • **Hamasaki, Hozumi**
 **Ibaraki-shi, Osaka-fu (JP)**
 • **Fujita, Tsuyoshi**
 **Ikeda-shi, Osaka-fu (JP)**

(74) Representative: **Harvey, David Gareth et al**
**Graham Watt & Co.**
**Riverhead**
**Sevenoaks Kent TN13 2BN (GB)**

(56) References cited:
 • BIOLOGICAL ABSTRACTS vol. 81, no. 11 , 1986, Philadelphia, PA, US; abstract no. 101243, HAND, D.; P. J. BUNGAY; B. .M. ELLIOTT AND M. GRIFFIN 'Activation of transglutaminase at calcium levels consistent with a role for this enzyme as a calcium receptor protein'
 • CLINICAL CHEMISTRY. vol. 31, no. 1, January 1985, WINSTON US pages 35 - 40 LASZLO MUSZBEK, JANOS POLGAR, AND LASZLO FES]S 'Kinetic Determination of Blood Coagulation Factor XIII in Plasma'
 • DATABASE WPIL Section Ch, Week 8712, Derwent Publications Ltd., London, GB; Class B04, AN 87-084135
 • DATABASE WPIL Section Ch, Week 8907, Derwent Publications Ltd., London, GB; Class B04, AN 89-051787
 • DATABASE WPIL Section Ch, Week 8943, Derwent Publications Ltd., London, GB; Class B04, AN 89-313512

**EP 0 555 046 B1**

## Description

This invention relates to a method for the determination of calcium and, more particularly, to a method of enzymatic determination of the amount of calcium in which calcium of a sample of blood serum is brought into contact with an apo-type transglutaminase (EC 2.3.2.13), which does not express the activity in the absence of calcium, thereby to determine the transglutaminase activity, which varies depending upon the amount of the calcium in the sample.

In accordance with the present invention removal of proteins from the sample is unnecessary and rapid and accurate determination of calcium in a sample is possible. Therefore, determination of calcium in samples such as human blood serum, is possible by the present invention, which therefore greatly contributes to clinical examinations, for example.

In a living body, 99 % or more of the calcium exists locally in the bones and teeth, and absorption and excretion of calcium into or from bones are repeated therein in an amount of about 700 mg/day. Therefore, though quantitatively smaller than in bones, calcium exists also in the body fluid and cells. Since calcium in the body fluid and cells participates not only in coagulation of blood but also in other various important actions of live activities, such as neurotransmitting function, myocontracting function and hormone function, the calcium level in the body fluid, especially in blood must be kept strictly constant. As well known, the calcium level in blood is kept constant due to vitamin D, accessory thyroid hormone, calcitonin and the like. In a healthy human body, calcium exists in blood in an amount of from 9 to 11 mg/dl, and the daily variation of the calcium level therein is strictly maintained to be at most +/- 3 %. However, when a person suffers from a disease, then its calcium level in blood noticeably fluctuates. For instance, the diseases of myxedema, malignant tumor, psychoidosis, hyperproteinemia, hyperthyroidism and the like cause hypercalcemia; while those of hypoparathyroidism, osteomalacia, renal rechitis, uremia, hypoproteinemia, osteotransfer of malignant tumor and the like cause hypocalcemia. Since the blood calcium level in a healthy person is very strictly maintained at a constant level, even slight fluctuations of the calcium level in a person may be judged to be surely caused by some disease with ease. Therefore, determination of the blood calcium is an extremely important test point for clinical examination.

For determination of the blood calcium, known are an atomic absorption method, an ionic electrode method and a chelate colorimetric method using OCPC (o-cresolphthalein complexon) or the like reagent. However, these methods involve various problems. For instance, an atomic absorption method and an ionic electrode method need complicated operations and particular and expensive instruments; and a chelate colorimetric method needs 8-hydroxyquinoline as a masking reagent since OCPC also reacts on magnesium due to the low reaction specificity thereof. 8-Hydroxyquinoline may mask magnesium to avoid a positive error, but it also traps calcium to cause other problems so that the coloring sensitivity is lowered and that calcium of low concentration may not be detected. In addition, the colorimetric method involves another problem that strict and severe control of the reagents to be used is necessary since the degree of coloration of the coloring system varies extremely due to slight fluctations of the pH value of the system.

Some methods of enzymatic determination of blood calcium are known, using an enzyme like the method of the present invention. For instance, JP-A 62-36199 (the term "JP-A" as used herein means an "unexamined published Japanese patent application") discloses a method of determining calcium in a sample, in which calcium in a sample is reacted with calmodulin to form a calcium/calmodulin complex and a calmodulin-dependent enzyme is activated with the resulting complex to measure the enzymatic activity thereof, thus to determine the calcium in the sample. However, the disclosed method involves various problems in that dilution of the sample to be tested is necessary since the detectable range is narrow and the sensitivity is high and in that the calmodulin and the substrate of the calmodulin-dependent enzyme to be used are expensive and unstable, so that it could hardly apply to various samples of a broad range. JP-A 64-2598 discloses a method of determining calcium in a sample, in which a calcium-containing sample is added to an excess oxalate and the amount of the oxalic acid as precipitated and retained in the reaction system is measured with an oxalic acid oxidase thereby to determine the calcium in the sample. However, the method is not satisfactory, since it needs an operation of precipitating calcium as calcium oxalate, it is influenced by ascorbic acid and bilirubin in the sample by using an oxalic acid oxidase for detection, it gives a standard curve of sloping downwards to the right side, and it cannot be apply to rate assays. JP-A 1-231896 discloses a method of determining calcium in a sample by measuring the enzymatic activity of phospholipase A2 as added to a calcium-containing sample, in which the enzymatic activity varies depending upon the calcium concentration in the sample in the presence of a substrate of a phosphorylcholine thioester. Since the method uses an SH-determining reagent such as DTNB (5,5'-dithiobis-2-nitrobenzoic acid) for detection of the enzymatic activity, it is directly influenced by sulfur-containing compounds such as cysteine and proteins in blood. A calcium determining method using an amylase has also been reported (Preprints in 23th General Meeting of Association for Promoting Automation of Clinical Examination of Japan, Address Nos. 222 and 223). As well known, however, an amylase also exists in blood and the content thereof largely fluctuates, depending upon the condition of diseases, individuals themselves and the time. Therefore, when a calcium concentration in a sample is determined on the basis of the amylase activity by the method, then the fluctuation of the blood amylase activity would give any positive or negative error to the calcium determination.

According to one aspect of the present invention, there is provided a method of determining the amount of calcium

2

in blood serum, comprising the steps of:

(1) providing a sample of blood serum;
(2) bringing blood serum calcium in said sample of blood serum into contact with a transglutaminase capable of being activated with calcium to provide an activated transglutaminase;
(3) allowing the activated transglutaminase to act on a substrate which is a mixture of a donor and an acceptor to generate $NH_3$;
(4) measuring the activity of transglutaminase by detecting the amount of $NH_3$ generated; and
(5) determining the amount of calcium in the sample of blood serum by correlating the amount of $NH_3$ generated with an amount of calcium.

According to another aspect of the present invention there is provided a method of determining the amount of calcium in blood serum, comprising the steps of:

(1) providing a sample of blood serum;
(2) bringing blood serum calcium in said sample of blood serum into contact with a transglutaminase capable of being activated with calcium to provide an activated transglutaminase;
(3) allowing the activated transglutaminase to act on a substrate which is a mixture of a donor and an acceptor, wherein said acceptor is hydroxylamine, to generate hydroxamic acid;
(4) measuring the activity of transglutaminase by detecting the amount of hydroxamic acid generated; and
(5) determining the amount of calcium in the sample of blood serum by correlating the amount of hydroxamic acid generated with an amount of calcium.

Preferred features of the invention are recited in dependent claims 3 to 9.
The invention will now be described by way of example only with reference to the accompanying drawings, in which:

Figure 1 shows a curve for determining calcium in an NADH system by the method of the present invention, and

Fig. 2 shows a curve for determining calcium in an NADPH system by the method of the present invention.

In consideration of the preceding problems, the present inventors earnestly studied and, as a result, have newly found that a transglutaminase (EC 2. 3. 2. 13) does not exist in blood serum and has an extremely high specificity to calcium and that the enzymatic activity of an apo-type transglutaminase varies depending upon the amount of calcium as added to the enzyme. On the basis of the findings, they have completed the present invention. Specifically, the present invention relates to enzyme assay of calcium, which is based upon a fundamental technical idea of bringing calcium in a sample into contact with a transglutaminase to measure the transglutaminase activity which varies, depending upon the amount of calcium in the sample, to thereby determine the amount of calcium in the sample.

Precisely, in accordance with the present invention, a sample to be tested is added to a reaction liquid at least containing an apo-type transglutaminase which does not express the activity in the absence of calcium, a substrate and a suitable buffer and detecting reagent so that the apo-type transglutaminase is converted to the corresponding holo-type one in proportion to the amount of calcium in the sample, whereupon the enzymatic activity is measured to thereby determine the amount of calcium in the sample.

A transglutaminase has a high specificity to calcium (Adv. Enzymol., 38, 109-191, 1973). Though the enzyme is activated with strontium, the km value of itself to strontium is larger than that to calcium by 20 times or more. Strontium is almost non-existent in body fluids such as blood or urine. Accordingly, the enzyme is said to be highly specific to calcium in clinical examination. Therefore, the present invention is completely free from the problem of magnesium, which is to be taken into consideration in the conventional OCPC method. In addition, a transglutaminase does not exist in serum. Although red corpuscles and platelets contain a blood coagulating factor XIII, which is grouped in the scope of a transglutaminase and which displays the same catalytic action as the enzyme. but they may be removed from serum in separating of blood clots therefrom. Therefore, the method of the present invention is free from the anxiety about positive or negative errors which are inevitable in the case of using an amylase. In accordance with the present invention, the transglutaminase activity is calculated from the amount of ammonia or hydroxamic acid to be formed by reaction with a substrate of glutamine peptide or glutamine peptide and amine. Where the activity is determined by detection of ammonia, it is to be noted that a large amount of ammonia is in urine. However, the presence of ammonia in urine causes no problem and determination of calcium in urine may well be effected by the method of the present invention, if a conventional isocitric acid dehydrogenase (ICDH) method (JP-A 61-247963, 62-6700, 62-6699) is applied thereto. Since the amount of ammonia in serum is slight, it. is therefore negligible. Accordingly, calcium in serum may well be determined by the method of the present invention, to which a conventional glutamic dehydrogenase (GIDH) may be applied. On the other hand, where the activity is determined by detection of hydroxamic acid, the method of the present invention involves no problem. Accordingly, the method of the present invention is superior to any other conventional enzymatic determination methods and is practical to use in daily clinical examinations, since the detection system is hardly influenced by the serum components and the reagents to be used are inexpensive.

The transglutaminase for use in the present invention may be any one extracted and purified from mammalian tissues, vegetables and microorganisms. As a preferred example of it, mentioned is one derived from livers of guinea pigs. For instance, the transglutaminase is obtained by disrupting livers of guinea pigs, subjecting the paste to centrifugation, and thereafter purifying the resulting supernatant by DEAE column chromatography, ammonium sulfate fractionation, gel permeation and hydrophobic chromatography. Preferred is the thus purified one having a high specificity to calcium with a small background. Any specific step is unnecessary for preparation of an apo-type transglutaminase, which may well be effected by merely adding a chelating agent to a buffer to be used in the purification step. If the purified enzyme has a high background, it has to be purified again or it is further subjected to a step of removing calcium therefrom. The morphology of the chelating reagent to be used is not specifically defined. The reagent is preferably one having a large chelating stability constant to calcium (for example, Log KML $\geq$ 10). Specifically mentioned are EDTA (ethylenediaminetetraacetic acid), CyDTA (trans-1,2-cyclohexanediamine-N,N,N',N'-tetraacetic acid), DTPA (diethylenetriamine-pentaacetic acid), GEDTA (glycol ether diamine-tetraacetic acid), TTHA (triethylenetetraminehexaacetic acid), methyl EDTA (diaminopropane-tetraacetic acid), etc. The chelating reagent is used in an amount of from 0.01 mM to 1 M, preferably from 0.1 mM to 100 mM, in such a way that the transglutaminase is not denatured by it. The chelating reagent is finally completely removed from the enzyme by dialysis or gel permeation using, for example, Sephadex (Registered Trade Mark) G-25, so that the enzyme may be used in the method of the present invention.

The transglutaminase reaction is as shown below.

$$X\text{-}L\text{-}Gln\text{-}Y \text{ (donor)} + R\text{-}NH_2 \text{ (acceptor)}$$

$$\rightarrow X\text{-}(L\text{-}Glu\text{-}NH\text{-}R)\text{-}Y + NH_3$$

The substrate to be added to the transglutaminase may be either in the form of a single donor or in the form of a combination of donor and an acceptor. In either case, the enzymatic activity of the transglutaminase is expressed. The transglutaminase has a high specificity to a donor, and various substrates of L-glutamic acid derivatives, tripeptides to higher polypeptides containing L-glutamine, and dipeptide to oligopeptide derivatives containing L-glutamine may be usable. Preferred substrates are benzyloxycarbonyl(Z)-L-Gln-Gly, Z-L-Gln, Z-L-Gln-L-Val, t-butyloxycarbonyl(Boc)-L-Gln, 9-fluorenylmethoxycarbonyl (Fmoc)-L-Gln, Gly-Gly-L-Gln-Gly, etc. On the other hand, since the transglutaminase has a low specificity to an acceptor, amines or hydroxylamines having three or more carbon chains, which are easily available and are inexpensive, are used as an acceptor. Preferably, the substrate for use in the present invention comprises, as a donor, Z-L-Gln-Gly, Z-L-Gln, Boc-L-Gln or Fmoc-L-Gln, and as an acceptor, n-propylamine, n-butylamine, n-amylamine, n-hexylamine, Lys or hydroxylamine. Combining a donor and an acceptor is preferred to the use of a donor only, as being able to give a larger enzymatic activity in the method of the present invention. Therefore, use of both a donor and an acceptor is recommended in the present invention, as a combined substrate.

For determination of the enzymatic activity in the method of the present invention, X-(L-Glu-NH-R)-Y or $NH_3$ to be formed by the reaction is detected since the transglutaminase catalyzes the preceding reaction. In the process, where a single donor or combination of a donor and an acceptor except hydroxylamine is used as the substrate, $NH_3$ formed is detected. On the other hand, where a combined substrate of a donor and hydroxylamine is used, X-(L-Glu-NHOH)-Y (hydroxamic acid) is detected. For detection of $NH_3$, any and every known method may be employed. As one preferred method, mentioned is a GIDH method. Needless to say, a different ICDH method, which has already been verified effective in various examination centers and which has been modified to have an ammonia-removing system, may also be employed for the purpose. As examples of the method, mentioned are those of using guanidinoacetic acid (JP-A 2-255098), urea (JP-A 61-247963) or creatinine (JP-A 59-31698). Detection of hydroxamic acid may also be effected by any known method. For instance, the method as described in J. Biol. Chem., 159, p. 21 (1954) is employable, in which the property of hydroxamic acid of forming a complex with $Fe^{3+}$ to give a red color is utilized and the absorbance of the complex at 525 nm is measured.

The method of the present invention will be explained in more detail by way of the following examples, which, however, are not intended to restrict the scope of the present invention.

Example 1:

Reagents:

First Reagent:

Z-Gln was dissolved in triethanolamine, and L-lysine, reduced glutathione, calcium chloride, aketoglutaric acid, ADP, NADH and glutamic dehydrogenase (derived from bovine liver) were added thereto. The resulting mix was adjusted to a pH of 7.5. Distilled water was added thereto to make the concentrations of the respective components 200 mM, 100 mM, 20 mM, 10 mM, 250 μm, 10 mM, 200 μm, 300 μm and 30 U/ml. The mix thus prepared is a first reagent.

Second Reagent:

Transglutaminase as purified from livers of guinea pigs was diluted with 10 mM tris-acetic acid buffer (pH 7) containing 2 mM of DTT to form an enzyme solution of 10 U/ml.

Samples:

Calcium chloride was dissolved and diluted in distilled water to give samples, having concentrations of 0 mM, 1 mM, 2 mM, 3 mM, 4 mM or 5 mM.

Process:

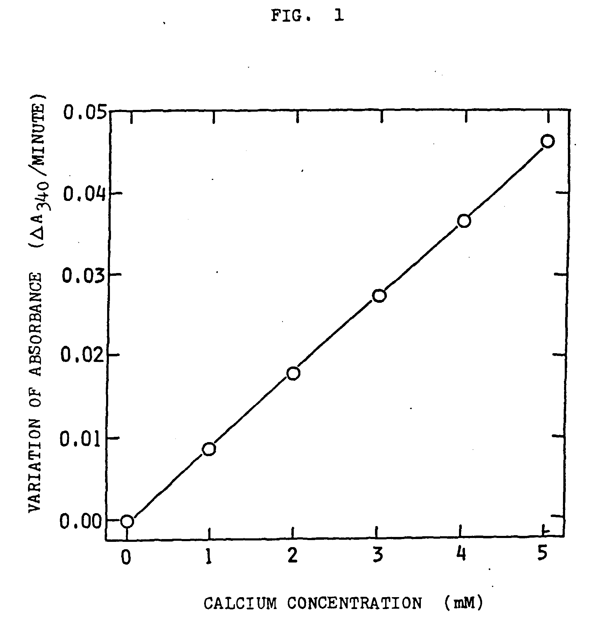

An automatic analyzer of Hitachi 7150 Model was used, in which the first reagent was put in R-1 and the second reagent in R-2. 10 μl of the sample was added to 200 μl of the first reagent and pre-incubated at 37°C for 5 minutes. Then, 40 μl of the second reagent was'added thereto. The reaction system was left as it was for 2 minutes at the same temperature after addition of the second reagent, and the deceleration of the absorbance of the system at 340 nm for one minute was measured. By subtracting the sample blank from the measured value, the result was drawn to give Fig. 1.

Result:

From the graph of Fig. 1, it is verified that the method of the present invention may determine the amount of calcium in a sample in the form of a linear quantitative determination curve.

Example 2:

Reagents:

First Reagent:

Z-Gln-Gly was dissolved in IM TRIS solution, and L-lysine, reduced glutathione, calcium chloride, α-ketoglutaric acid, NADH and glutamic dehydrogenase (derived from yeast) were added thereto. The resulting mix was adjusted to a pH of 9 with NaOH. Distilled water was added thereto to make the concentrations of the respective components to be 30 mM, 200 mM, 100 mM, 10 mM, 150 μM, 10 mM, 250 μm and 10 U/ml. The mix thus prepared is a first reagent.

Second Reagent:

This is same as that in Example 1.

Samples:

These are same as those in Example 1.

Process:

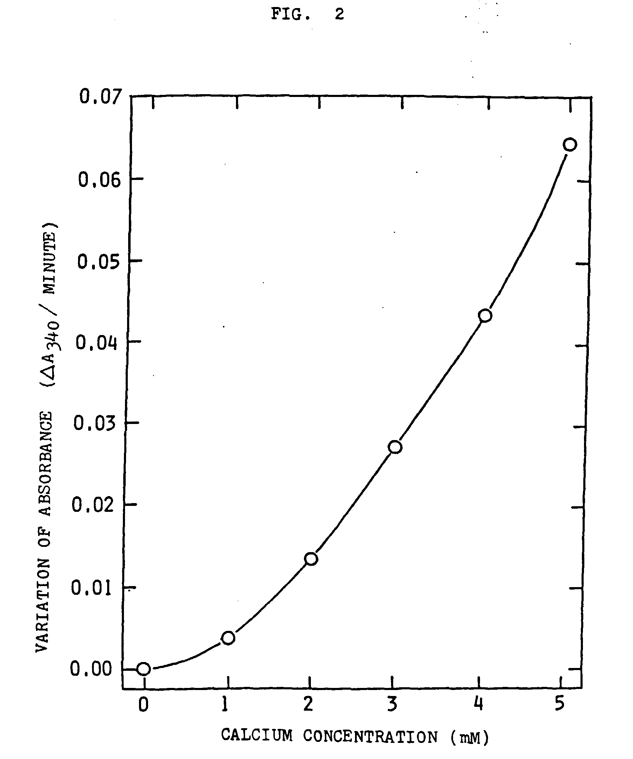

This is same as that in Example 1. The result was drawn to give Fig. 2.

Result:

As is noted in Fig. 2, the transglutaminase activity in the samples varies, depending upon the calcium concentration in them, in accordance with the method of the present invention. Therefore, it is verified that the calcium concentration in the respective samples is well determined by the method of the present invention.

The method of the present invention needs no operation for removal of proteins from a sample of blood serum to be tested by it. In accordance with the method, therefore, simple and rapid determination of calcium in the sample is possible. Accordingly, rapid and accurate determination of calcium may be effected by the method of the present invention without any skill of carrying out the method. Thus, the present invention greatly contributes to, for example, clinical diagnoses needing determination of calcium content.

**Claims**

1. A method of determining the amount of calcium in blood serum, comprising the steps of:

   (1) providing a sample of blood serum;
   (2) bringing blood serum calcium in said sample of blood serum into contact with a transglutaminase capable of being activated with calcium to provide an activated transglutaminase;
   (3) allowing the activated transglutaminase to act on a substrate which is a mixture of a donor and an acceptor to generate $NH_3$;
   (4) measuring the activity of transglutaminase by detecting the amount of $NH_3$ generated; and
   (5) determining the amount of calcium in the sample of blood serum by correlating the amount of $NH_3$ generated with an amount of calcium.

2. A method of determining the amount of calcium in blood serum, comprising the steps of:

   (1) providing a sample of blood serum;
   (2) bringing blood serum calcium in said sample of blood serum into contact with a transglutaminase capable of being activated with calcium to provide an activated transglutaminase;
   (3) allowing the activated transglutaminase to act on a substrate which is a mixture of a donor and an acceptor, wherein said acceptor is hydroxylamine, to generate hydroxamic acid;
   (4) measuring the activity of transglutaminase by detecting the amount of hydroxamic acid generated; and
   (5) determining the amount of calcium in the sample of blood serum by correlating the amount of hydroxamic acid generated with an amount of calcium.

3. A method according to claim 1, wherein said donor is a compound represented by the formula,

$$X-L-Gln-Y,$$

   wherein X is an amino acid, peptide or protective group at the N-terminal end of L-Gln, and Y is an amino acid peptide or hydrogen atom at the C-terminal end of L-Gln, and said acceptor is a compound represented by the formula:

$$R-NH_2,$$

   wherein R is $(CH_2)_nCH_3$ where n=2-5, OH or lysine residue ($R-NH_2$=lysine).

4. A method according to claim 2, wherein said donor is a compound represented by the formula,

$$X-L-Gln-Y,$$

   wherein X is an amino acid, peptide or protective group at the N-terminal end of L-Gln, and Y is an amino acid, peptide or hydrogen atom at the C-terminal end of L-Gln.

5. A method according to claim 1 or claim 3, wherein said donor is selected from the group consisting of benzyloxycarbonyl-L-Gln-Gly, benzyloxycarbonyl-L-Gln, t-butyloxycarbonyl-L-Gln, and 9-fluorenylmethoxycarbonyl-L-Gln, and said acceptor is selected from the group consisting of n-propylamine, n-butylamine, n-amylamine, n-hexylamine, and lysine.

6. A method according to claim 2, wherein said donor is selected from the group consisting of benzyloxycarbonyl-L-Gln-Gly, benzyloxycarbonyl-L-Gln, t-butyloxycarbonyl-L-Gln, and 9-fluorenylmethoxycarbonyl-L-Gln.

7. A method according to claim 1 or claim 2, further comprising the step of pre-incubating said substrate about 37°C prior to activating said transglutaminase.

8. A method according to claim 1 or claim 2, wherein step (2) is carried out at a temperature of about 37°C for about two minutes.

9. A method according to claim 7, wherein said transglutaminase is added to said sample for two minutes in an amount of about 10 U/ml prior to step 4.

**Patentansprüche**

1. Verfahren zur Bestimmung der Calciummenge in Blutserum, bestehend aus oder enthaltend die Schritte:

   (1) Bereitstellen einer Blutserumprobe,
   (2) Kontaktieren des Blutserumcalciums in der Blutserumprobe mit durch Calcium aktivierbarer Transglutaminase zur Bereitstellung einer aktivierten Transglutaminase,
   (3) Einwirkenlassen der aktivierten Transglutaminase auf ein Substrat, das ein Gemisch aus einem Spender und einem Akzeptor ist, zur Bildung von $NH_3$,
   (4) Messen der Transglutaminaseaktivität durch Bestimmung der gebildeten $NH_3$-Menge und
   (5) Bestimmung der Calciummenge in der Blutserumprobe durch Korrelation der gebildeten $NH_3$-Menge mit einer Menge Calcium.

2. Verfahren zur Bestimmung der Calciummenge in Blutserum, bestehend aus oder enthaltend die Schritte:

   (1) Bereitstellen einer Blutserumprobe,
   (2) Kontaktieren des Blutserumcalciums in der Blut-serumprobe mit durch Calcium aktivierbarer Transglutaminase zur Bereitstellung einer aktivierten Transglutaminase,
   (3) Einwirkenlassen der aktivierten Transglutaminase auf ein Substrat, das aus einem Gemisch aus einem Spender und einem Akzeptor besteht, wobei der Akzeptor Hydroxylamin ist, zur Bildung von Hydroxamsäure,
   (4) Messen der Transglutaminaseaktivität durch Bestimmung der gebildeten Hydroxamsäure und
   (5) Bestimmung der Calciummenge in der Blutserumprobe durch Korrelation der gebildeten Hydroxamsäuremenge zu einer Menge Calcium

3. Verfahren gemäß Anspruch 1, wobei der Spender eine durch die Formel

$$X\text{-}L\text{-}Gln\text{-}Y$$

dargestellte Verbindung ist,
worin X eine Aminosäure, ein Peptid oder eine Schutzgruppe am N-Ende von L-Gln und Y ein Aminosäurepeptid oder Wasserstoffatom am C-Ende von L-Gln ist,
und der Akzeptor eine durch die Formel

$$R\text{-}NH_2$$

dargestellte Verbindung ist,
worin R $(CH_2)_n CH_3$ mit n = 2 bis 5, OH oder ein Lysinrest ($R\text{-}NH_2$ = Lysin) bedeutet.

4. Verfahren gemäß Anspruch 2, wobei der Spender eine durch die Formel

X-L-Gln-Y

dargestellte Verbindung ist,
worin X eine Aminosäure, ein Peptid oder eine Schutzgruppe am N-Ende von L-Gln und Y eine Aminosäure, ein Peptid oder Wasserstoffatom am C-Ende von L-Gln ist.

5. Verfahren gemäß Anspruch 1 oder Anspruch 3, wobei der Spender ausgewählt ist aus der Gruppe bestehend aus Benzyl-oxycarbonyl-L-Gln-Gly, Benzyloxycarbonyl-L-Gln,t-Butyloxycarbonyl-L-Gln und 9-Fluorenylmethoxycarbonyl-L-Gln, und der Akzeptor ausgewählt ist aus der Gruppe bestehend aus N-Propylamin, N-Butylamin, N-Amylamin, N-Hexylamin und Lysin.

6. Verfahren gemäß Anspruch 2, wobei der Spender ausgewählt ist aus der Gruppe bestehend aus Benzyloxycarbonyl-L-Gln-Gly, Benzyloxycarbonyl-L-Gln, t-Butyloxycarbonyl-L-Gln und 9-Fluorenylmethoxycarbonyl-L-Gln.

7. Verfahren gemäß Anspruch 1 oder Anspruch 2, außerdem enthaltend den Schritt der Präinkubation des Substrats bei etwa 37 °C vor Aktivierung der Transglutaminase.

8. Verfahren gemäß Anspruch 1 oder Anspruch 2, wobei Schritt (2) etwa zwei Minuten bei einer Temperatur von etwa 37 °C durchgeführt wird.

9. Verfahren gemäß Anspruch 7, wobei vor Schritt 4 die Transglutaminase der Probe zwei Minuten in einer Menge von etwa 10 U/ml zugegeben wird.


**Revendications**

1. Procédé de détermination de la quantité de calcium dans du sérum sanguin, comprenant les étapes consistant :

   (1) à préparer un échantillon de sérum sanguin ;
   (2) à mettre en contact le calcium de sérum sanguin présent dans ledit échantillon de sérum sanguin, avec une transglutaminase capable d'être activée avec le calcium, pour obtenir une transglutaminase activée ;
   (3) à laisser la transglutaminase activée, agir sur un substrat consistant en un mélange d'un donneur et d'un accepteur afin de générer $NH_3$ ;
   (4) à mesurer l'activité de la transglutaminase, par détermination de la quantité de $NH_3$ généré ; et
   (5) à déterminer la quantité de calcium dans l'échantillon de sérum sanguin, par corrélation de la quantité de $NH_3$ généré, avec une quantité de calcium.

2. Procédé de détermination de la quantité de calcium dans du sérum sanguin, comprenant les étapes consistant :

   (1) à préparer un échantillon de sérum sanguin ;
   (2) à mettre le calcium de sérum sanguin présent dans l'échantillon de sérum sanguin, en contact avec une transglutaminase capable d'être activée avec le calcium, pour obtenir une transglutaminase activée ;
   (3) à laisser la transglutaminase activée, agir sur un substrat consistant en un mélange d'un donneur et d'un accepteur, l'accepteur étant l'hydroxylamine, afin de générer de l'acide hydroxamique ;
   (4) a mesurer l'activité de la transglutaminase, par détermination de la quantité d'acide hydroxamique généré ; et
   (5) à déterminer la quantité de calcium présent dans l'échantillon de sérum sanguin, par corrélation de la quantité d'acide hydroxyamique généré avec une quantité de calcium.

3. Procédé selon la revendication 1, dans lequel le donneur est un composé représenté par la formule :

X-L-Gln-Y,

dans laquelle X représente un aminoacide, un peptide ou un groupe protecteur à l'extrémité N-terminale de L-Gln, et Y représente un peptide d'aminoacide ou un atome d'hydrogène, à l'extrémité C-terminale de L-Gln, et cet accepteur étant un composé représenté par la formule :

$$R-NH_2,$$

dans laquelle R représente $(CH_2)_nCH_3$, n variant de 2 à 5, un groupe OH ou un résidu lysine ($R-NH_2$=lysine).

4. Procédé selon la revendication 2, dans lequel le donneur est un composé représenté par la formule :

$$X-L-Gln-Y,$$

dans laquelle X représente un aminoacide, un peptide ou un groupe protecteur au niveau de l'extrémité N-terminale de L-Gln, et Y représente un aminoacide, un peptide ou un atome d'hydrogène au niveau de l'extrémité C-terminale de L-Gln.

5. Procédé selon la revendication 1 ou 3, dans lequel le donneur est choisi parmi les benzyloxycarbonyl-L-Gln-Gly, benzyloxycarbonyl-L-Gln, t-butyloxycarbonyl-L-Gln et 9-fluorénylméthoxycarbonyl-L-Gln, et l'accepteur est choisi parmi la n-propylamine, la n-butylamine, la n-amylamine, la n-hexylamine et la lysine.

6. Procédé selon la revendication 2, dans lequel le donneur est choisi parmi les benzyloxycarbonyl-L-Gln-Gly, benzyloxycarbonyl-L-Gln, t-butyloxycarbonyl-L-Gln, et 9-fluorénylméthoxycarbonyl-L-Gln.

7. Procédé selon la revendication 1 ou 2, comprenant en outre l'opération de pré-incubation du substrat à une température d'environ 37 °C avant l'activation de la transglutaminase.

8. Procédé selon la revendication 1 ou 2, dans lequel l'opération (2) est effectuée à une température d'environ 37 °C pendant environ deux minutes.

9. Procédé selon la revendication 7, dans lequel la transglutaminase est ajoutée dans l'échantillon, pendant deux minutes, selon une quantité d'environ 10 U/ml avant l'opération 4.

FIG. 1

FIG. 2